# EUROPEAN PATENT APPLICATION

(11) **EP 4 825 834 A1**
(43) Date of publication of application: **21.10.2026**
(21) Application number: 25808454.0
(22) Date of filing: 05.03.2025
(51) Int. Cl.: C07B 59/00, C07D 207/16, C07C 229/22, C07C 229/08, C07K 5/062, C07K 1/107

(54) **METHOD FOR EFFECTIVE ENANTIOSELECTIVE DEUTERATION OF alpha-HYDROGEN OF alpha-AMINO ACIDS WITHOUT EXTERNAL CHIRAL SOURCE**

(30) Priority: 21.05.2024 KR 20240065591; 04.12.2024 KR 20240178352
(71) Applicant: Seoul National University R&DB Foundation, Seoul 08826 (KR)
(72) Inventor: KIM, Sang Hee, Seoul 08826 (KR); PARK, Soo Jun, Seoul 08826 (KR); KIM, Dong Jun, Seoul 08826 (KR)
(74) Representative: TRBL Intellectual Property
(86) International application number: PCT/KR2025/099571
(87) International publication number: WO 2025/244517

(57) **Abstract**

The present invention relates to a method for asymmetrically deuterating hydrogen at the α-position in an α-amino acid derivative, and specifically may include, preparing an α-amino acid derivative including a structure of Chemical Formula 1; treating the α-amino acid derivative with a base in a solvent to induce a deuteration reaction and generating a product; and purifying the product. 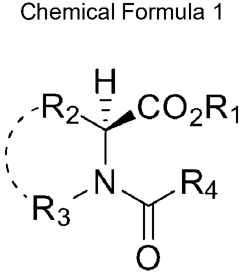 (R₁ includes one or more of alkyl, alkenyl, alkynyl, or hydrogen, R₂ and R₃ include one or more of alkyl, -C(O)alkyl, alkenyl, -C(O)alkenyl, cycloalkyl, -C(O)cycloalkyl, cycloalkenyl, -C(O)cycloalkenyl, aryl, -C(O)aryl, heteroaryl, -C(O)heteroaryl, heterocyclyl, or -C(O)heterocyclyl, R₄ includes one or more of alkyl, alkenyl, alkynyl, or hydrogen)

## Description

### TECHNICAL FIELD

The present invention relates to a method for deuterating amino acids.

### BACKGROUND ART

Deuterated α-amino acids are considered important in the fields of drug discovery and biomedical science, since they have a kinetic isotope effect (KIE). For example, deuterated α-amino acids are used in research for elucidating secondary and quaternary structures of proteins, or for investigating biochemical processes such as the metabolism of peptides and enzyme mechanisms. In particular, α-amino acids in which deuterium is substituted at the α-position are attracting attention because they may show improved metabolic stability compared to a general α-amino acid moiety.

In addition, since chirality of α-amino acids has a great influence on biological activity, a chirality-selective deuteration technique has greater value than a simple deuteration technique. However, in the process of substituting hydrogen at the α-position with deuterium, a double bond is formed and there is a possibility that chirality may be lost, and thus great difficulty is involved in implementing this technique at a high level. For this reason, various methodologies for efficiently producing chirally deuterated α-amino acids have been proposed.

However, conventional techniques have several clear limitations. Methods using an external chiral source (chiral metal complex and organocatalyst) or enzyme-based techniques have a limited range of applicable substrates (scope), and the process economy is low. In addition, methods for asymmetrically deuterating the α-position after introducing a specific protecting group into an α-amino acid require an anhydrous condition, an extremely low temperature (-78°C), and the use of a strong base, and thus have low practicality in a large-scale process.

Therefore, there is an urgent need to develop a new method capable of increasing convenience of a process, maintaining chirality, and effectively substituting hydrogen at the α-position with deuterium.

### BRIEF DESCRIPTION OF THE INVENTION

### PROBLEM TO BE SOLVED

The present invention is intended to solve the above-described conventional problems, and an object of the present invention is to provide a method for chirality-selectively substituting hydrogen at the α-position of an α-amino acid with deuterium even without introducing an external chiral source.

Another object of the present invention is to provide a technique capable of effectively deuterating hydrogen at the α-position while maintaining chirality of an amino acid, even without requiring extreme conditions (for example, an extremely low temperature, an anhydrous environment, a strong base, etc).

### MEANS FOR SOLVING THE PROBLEM

An asymmetric α-deuteration method of an α-amino acid derivative according to one embodiment of the present invention may include, preparing an α-amino acid derivative including a structure of Chemical Formula 1; treating the α-amino acid derivative with a base in a solvent to induce a deuteration reaction and generating a product; and purifying the product. 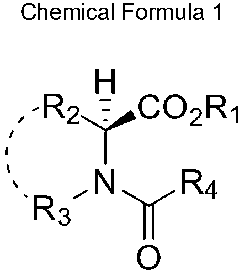 R₁ includes one or more of alkyl, alkenyl, alkynyl, or hydrogen, R₂ and R₃ include one or more of alkyl, -C(O)alkyl, alkenyl, -C(O)alkenyl, cycloalkyl, -C(O)cycloalkyl, cycloalkenyl, - C(O)cycloalkenyl, aryl, -C(O)aryl, heteroaryl, -C(O)heteroaryl, heterocyclyl, or - C(O)heterocyclyl, R₄ includes one or more of alkyl, alkenyl, alkynyl, or hydrogen)

According to one embodiment, the method may be substituting hydrogen at the α-position with deuterium while maintaining chirality at the α-position of the α-amino acid derivative.

According to one embodiment, the α-amino acid derivative may include an N-protecting group substituted on N.

According to one embodiment, the solvent includes one or more selected from the group consisting of a C₁ to C₁₀ alcohol-based solvent, acetonitrile, dimethyl sulfoxide (DMSO), acetone, ether and cyclic ether, nitromethane, chloroform, dimethylformamide (DMF), toluene, and water (H₂O), and the solvent may include at least one deuterium atom in a molecular structure to promote the deuteration reaction.

According to one embodiment, the base may include a cation and alkoxide (MOR).

According to one embodiment, the method may further include, between the step of generating the product; and the step of purifying the product; stopping the deuteration reaction; and the step of stopping the deuteration reaction may be treating the product with a weakly acidic solution.

According to one embodiment, the step of purifying the product may be purifying the product through one or more methods selected from the group consisting of chromatography, crystallization, extraction, distillation, recrystallization, filtration, evaporation, sublimation, fractional extraction, washing, and the like.

According to one embodiment, the method may further include, after the step of purifying the product; removing an N-protecting group of the deuterated α-amino acid derivative included in the purified product.

An α-amino acid derivative including deuterium at the α-position according to another embodiment may include a structure of Chemical Formula 2, and Chemical Formula 2 may be prepared by the asymmetric α-deuteration method of an α-amino acid derivative according to one embodiment. 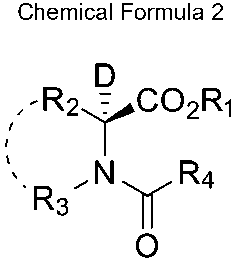 (R₁ includes one or more of alkyl, alkenyl, alkynyl, or hydrogen, R₂ and R₃ include one or more of alkyl, -C(O)alkyl, alkenyl, -C(O)alkenyl, cycloalkyl, -C(O)cycloalkyl, cycloalkenyl, - C(O)cycloalkenyl, aryl, -C(O)aryl, heteroaryl, -C(O)heteroaryl, heterocyclyl, or - C(O)heterocyclyl, R₄ includes one or more of alkyl, alkenyl, alkynyl, or hydrogen)

According to one embodiment, the α-amino acid derivative may be a proline derivative in which hydrogen at the α-position is deuterated.

According to one embodiment, the deuteration level (DL) of the α-amino acid may be 72% or more.

According to one embodiment, the enantiomeric excess (EE) of the α-amino acid may be 90% or more.

### EFFECTS OF THE INVENTION

The present invention has an effect of providing a method for deuterating hydrogen at the α-position of an amino acid without introducing an external chiral source and without extreme environmental conditions.

In addition, the deuteration method of the present invention has an effect that chirality of the deuterated α-amino acid can be maintained.

However, the effects of the present invention are not limited to the effects described above, and include all effects naturally implemented due to various configurations proposed in the present invention.

### DESCRIPTION OF THE DRAWINGS

FIG. 1 is a flowchart illustrating an asymmetric α-deuteration method of an α-amino acid derivative according to one embodiment of the present invention.
FIG. 2 is a chemical reaction formula illustrating a method for preparing an α-amino acid derivative including a structure of Chemical Formula 1.
FIG. 3 is a chemical reaction formula illustrating a method for asymmetrically α-deuterating a proline derivative and a drawing illustrating results thereof.
FIG. 4 is a table illustrating results of performing a deuteration reaction of a proline derivative under various conditions.
FIG. 5 is formula (a) illustrating a plausible reaction pathway of the asymmetric α-deuteration method for a proline derivative and a graph (b) illustrating a free energy profile.
FIG. 6 is a drawing illustrating results of deuteration reactions of various proline derivatives.
FIG. 7 is a drawing illustrating results of deuteration reactions of pseudoproline derivatives.
FIG. 8 is a drawing illustrating results of deuteration reactions of acyclic α-amino acid derivatives.
FIG. 9 is a drawing illustrating a step of removing an N-protecting group of a deuterated α-amino acid derivative.

### DETAILED DESCRIPTION OF THE INVENTION

Embodiments of the present invention are exemplified for the purpose of explaining the technical idea of the present invention. The scope of rights according to the present invention is not limited to the embodiments presented below or the detailed description of these embodiments.

All technical terms and scientific terms used in the present invention, unless otherwise defined, have meanings generally understood by a person having ordinary skill in the technical field to which the present invention pertains. All terms used in the present invention are selected for the purpose of more clearly describing the present invention, and are not selected to limit the scope of rights according to the present invention.

Expressions such as "comprising", "including", "having", and the like used in the present invention should be understood as open-ended terms implying the possibility of including other embodiments, unless otherwise mentioned in a phrase or sentence in which the corresponding expression is included.

A singular expression described in the present invention may include a plural meaning unless otherwise mentioned, and this is equally applied to a singular expression described in the claims.

An asymmetric α-deuteration method of an α-amino acid derivative according to one embodiment of the present invention may include, preparing an α-amino acid derivative including a structure of Chemical Formula 1; treating the α-amino acid derivative with a base in a solvent to induce a deuteration reaction and generating a product; and purifying the product.

According to one embodiment, the method may be substituting hydrogen at the α-position with deuterium while maintaining chirality at the α-position of the α-amino acid derivative.

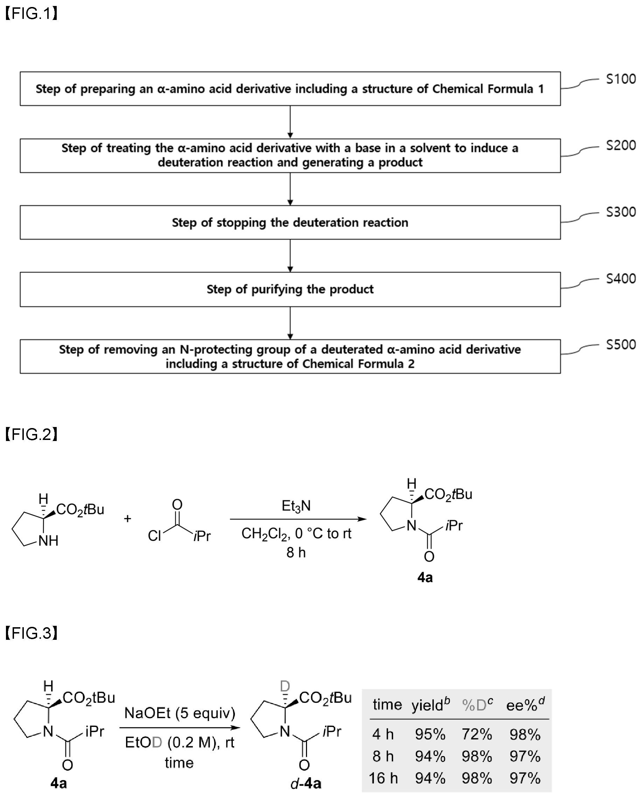

FIG. 1 is a flowchart illustrating an asymmetric α-deuteration method of an α-amino acid derivative according to one embodiment of the present invention.

The present invention proposes a new method capable of obtaining an α-deuterated α-amino acid derivative by substituting hydrogen at the α-position with deuterium while maintaining chirality at the α-position of an α-amino acid derivative, and consequently obtaining an α-deuterated amino acid in which chirality is maintained. In particular, the present invention does not require extreme conditions, catalysts, and the like, unlike conventional techniques and thus process convenience is improved, and the scope of substrates to which the deuteration method can be applied is expanded compared to conventional techniques, and has an effect that chirality of the α-amino acid can be maintained even after the deuteration reaction. An α-amino acid may be obtained from the amino acid derivative in which hydrogen at the α-position has been substituted with deuterium by the method described herein.

Hereinafter, each step of the asymmetric α-deuteration method of an α-amino acid derivative is described.

### 1. Step of preparing an α-amino acid derivative including a structure of Chemical Formula 1 (S100)

The method is a method of substituting hydrogen with deuterium while maintaining chirality at the α-position of the α-amino acid derivative, and the α-amino acid derivative may include a structure of Chemical Formula 1. 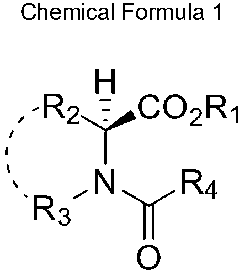 (R₁ includes one or more of alkyl, alkenyl, alkynyl, or hydrogen, R₂ and R₃ include one or more of alkyl, -C(O)alkyl, alkenyl, -C(O)alkenyl, cycloalkyl, -C(O)cycloalkyl, cycloalkenyl, - C(O)cycloalkenyl, aryl, -C(O)aryl, heteroaryl, -C(O)heteroaryl, heterocyclyl, or - C(O)heterocyclyl, R₄ includes one or more of alkyl, alkenyl, alkynyl, or hydrogen)

According to one embodiment, the α-amino acid derivative may include an *N*-protecting group substituted on N.

In the case of an α-amino acid derivative not substituted with the *N*-protecting group, the chirality at the α-position may not be maintained. Therefore, by substituting N of the α-amino acid derivative with a bulky protecting group having large steric hindrance, axial chirality may be imparted to a double bond formed during a deuteration process, and the chirality at the α-position may be maintained.

The *N*-protecting group may include one or more selected from the group consisting of isobutyryl, pivaloyl, and tosyl including an electron withdrawing group (EWG) such as carbonyl, sulfonyl, sulfoxide, and the like.

The α-amino acid derivative including the structure of Chemical Formula 1 may be prepared in a solution state in a solvent.

According to one embodiment, the solvent may include one or more selected from the group consisting of a C₁ to C₁₀ alcohol-based solvent, acetonitrile, dimethyl sulfoxide (DMSO), acetone, ether and cyclic ether, nitromethane, chloroform, dimethylformamide (DMF), toluene, and water (H₂O), and the solvent may include deuterium. The solvent may be used as long as it is a solvent capable of dissolving the derivative, and is not particularly limited in the present invention.

The solvent may be included such that the α-amino acid derivative has a concentration of 0.01 mol/L to 2.0 mol/L. When the concentration exceeds 2.0 mol/L, there may be a problem in that process efficiency is reduced and a yield is reduced.

In one example, the solvent may serve to provide deuterium capable of being substituted at the α-position of the α-amino acid derivative when a deuteration reaction is subsequently induced, by including deuterium in a molecular structure. Through this, the deuteration reaction may proceed. The solvent may preferably include a C₁ to C₁₀ alcohol-based solvent. In one example, the solvent may be ethanol (EtOD) including deuterium.

### 2. Step of treating the α-amino acid derivative with a base in a solvent to induce a deuteration reaction and generating a product (S200)

The step of generating the product may be adding a base to a solution including the α-amino acid derivative and a solvent including deuterium and stirring the solution.

Since the base serves to attack and remove hydrogen positioned at the α-position of the α-amino acid derivative, deuterium included in the solvent may be substituted at the α-position.

According to one embodiment, the base may include a cation and alkoxide (MOR). In one example, the base may be sodium ethoxide (NaOEt), lithium ethoxide (LiOEt), or potassium ethoxide (KOEt), but is not limited thereto.

The base may be added in an amount of 1 equivalent (eq.) to 10 equivalents (eq.) with respect to the amino acid. When the base is included in an amount of less than 1 equivalent with respect to the amino acid, a rate of deuteration is slow, and when the base is included in an amount of more than 10 equivalents, a yield may be reduced due to an ester exchange reaction. Preferably, the base may be added in an amount of 1 equivalent (eq.) to 5 equivalents (eq.) with respect to the amino acid.

The deuteration reaction may be performed at -78.5°C to 30°C, and preferably may be performed under room temperature conditions of 0°C to 25°C. Unlike conventional techniques, since the deuteration method of the present invention may be performed under room temperature conditions rather than extremely low temperature conditions, there is an effect that convenience of a process is greatly improved.

The deuteration reaction may be performed for 30 minutes to 170 hours.

An α-amino acid derivative including deuterium at the α-position according to another embodiment may include a structure of Chemical Formula 2, and Chemical Formula 2 may be prepared by the asymmetric α-deuteration method of an α-amino acid derivative according to one embodiment. 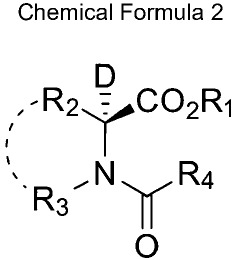 (R₁ includes one or more of alkyl, alkenyl, alkynyl, or hydrogen, R₂ and R₃ include one or more of alkyl, -C(O)alkyl, alkenyl, -C(O)alkenyl, cycloalkyl, -C(O)cycloalkyl, cycloalkenyl, - C(O)cycloalkenyl, aryl, -C(O)aryl, heteroaryl, -C(O)heteroaryl, heterocyclyl, or - C(O)heterocyclyl, R₄ includes one or more of alkyl, alkenyl, alkynyl, or hydrogen)

Through the above-described asymmetric α-deuteration method of an α-amino acid derivative, an α-amino acid derivative in which hydrogen at the α-position is substituted with deuterium may be obtained. As can be confirmed in Chemical Formula 2, the hydrogen at the α-position of the α-amino acid derivative of Chemical Formula 1 is substituted with deuterium in the α-amino acid derivative of Chemical Formula 2, and the chirality at the α-position of the α-amino acid derivative may be maintained.

Therefore, through the α-deuteration method of the present invention, an α-amino acid in which chirality is maintained may be more simply prepared than in conventional techniques, and there may be an advantage that a range of the scope of substrates to which the method can be applied is wider.

### 3. Step of stopping the deuteration reaction (S300)

According to one embodiment, the method may further include, between the step of generating the product; and the step of purifying the product; stopping the deuteration reaction; and the step of stopping the deuteration reaction may be treating the product with a weakly acidic solution.

The step of stopping the deuteration reaction is for stopping the deuteration reaction after performing the deuteration reaction at a predetermined temperature and time. The weakly acidic solution may stop (quench) the deuteration reaction by neutralizing the base, by providing a proton. In one example, the weakly acidic solution may be a saturated ammonium chloride (NH₄Cl) aqueous solution or acetic acid (CH₃COOH), but is not limited thereto.

After stopping the deuteration reaction, the solvent may be removed under reduced pressure. After adding water and an appropriate organic solvent to the reaction product from which the solvent has been removed, an organic fraction may be extracted, and the organic fraction may be extracted with one or more selected from the group consisting of ethyl acetate (EtOAc), hexane, cyclohexane, toluene, benzene, acetone, acetonitrile, petroleum ether, dichloromethane (DCM), 1,2-dichloroethane (DCE), methanol (MeOH), ethanol (EtOH), isopropanol (IPA), and chloroform.

### 4. Step of purifying the product (S400)

According to one embodiment, the step of purifying the product may be purifying the product through one or more methods selected from the group consisting of chromatography, crystallization, extraction, distillation, recrystallization, filtration, evaporation, sublimation, fractional extraction, washing, and the like.

The step of purifying may be performed to remove impurities from the product obtained by the above-described method.

The extracted organic fraction may be dried and concentrated under reduced pressure. In one example, finely remaining water may be removed by treating the organic fraction with magnesium sulfate (MgSO₄), and the organic fraction may be dried by removing the solvent under reduced pressure.

In one example, the step of purifying may be purifying the concentrated product through flash chromatography. After performing the step of purifying, the deuterated α-amino acid derivative may be obtained.

A deuteration level at the α-position of the obtained α-amino acid derivative may be measured through ¹H NMR analysis and liquid chromatography mass spectrometry (LC/MS).

### 5. Step of removing an N-protecting group of a deuterated α-amino acid derivative (S500)

According to one embodiment, the method may further include, after the step of purifying the product; removing an *N*-protecting group of the deuterated α-amino acid derivative included in the purified product.

The deuterated α-amino acid may be obtained by removing a protecting group, such as a carbonyl *N*-protecting group, included in the α-amino acid derivative.

A method for removing the *N*-protecting group of the α-amino acid derivative is not particularly limited in the present invention, and may be performed through a conventionally known reduction reaction and reactions generally known to be available. In one example, the step of removing the *N*-protecting group may be performed by adding hydrochloric acid (HCl).

The method for removing the *N*-protecting group of the α-amino acid derivative may be performed in a plurality of steps, and is not limited as long as it is a method capable of consequently obtaining a deuterated α-amino acid.

After removing the *N*-protecting group as described above, the α-amino acid including deuterium at the α-position and maintaining chirality may be obtained.

Hereinafter, the present invention is described in more detail through embodiments. However, these embodiments show some experimental methods and configurations for exemplarily describing the present invention, and the scope of the present invention is not limited to these embodiments.

### Preparation Embodiment 1: Preparation of proline derivative including a structure of Chemical Formula 1

In order to prepare the α-amino acid derivative including the above-described structure of Chemical Formula 1, an amino acid proline derivative was used.

FIG. 2 is a chemical reaction formula illustrating a method for preparing an α-amino acid derivative including a structure of Chemical Formula 1.

Et₃N (24.4 mL, 175.2 mmol, 3.0 equiv) and isobutyryl chloride (6.73 mL, 64.2 mmol, 1.1 equiv) were added at 0°C to *tert*-butyl L-prolinate (10.0 g, 58.4 mmol, 1.0 equiv) included in CH₂Cl₂ solvent (195 mL). The mixed solution was stirred at room temperature for 8 hours. After stirring, the mixed solution was cooled to 0°C, and saturated NH₄Cl aqueous solution (40 mL) was added to stop the reaction. Thereafter, 100 mL of water was added, and extraction was performed three times (3 × 100 mL) with CH₂Cl₂. The extracted organic fraction was dried using MgSO₄ and concentrated under reduced pressure.

After concentration, purification was performed by flash chromatography (hexane/EtOAc=3:1, v/v) on silica gel, and a proline derivative *tert*-butyl isobutyryl-L-prolinate (4a) including Chemical Formula 1 in the form of colorless oil was obtained (14.0 g, 99%).

### Experimental Embodiment 1: Deuteration reaction of proline derivative

A deuteration reaction was performed by treating the proline derivative (4a, 0.2 mmol) obtained in Preparation Embodiment 1 with NaOEt (5.0 equiv) and EtOD (0.2 M) at room temperature,

FIG. 3 is a chemical reaction formula illustrating a method for asymmetrically α-deuterating a proline derivative and a drawing illustrating results thereof.

According to one embodiment, the α-amino acid derivative may be a proline derivative in which hydrogen at the α-position is deuterated.

By the deuteration reaction of the chemical reaction formula, the proline derivative (4a) is transformed into a proline derivative (d-4a) in which hydrogen at the α-position is substituted with deuterium, and it can be confirmed through HPLC that chirality at the α-position is maintained.

According to one embodiment, the deuteration level (DL) of the α-amino acid derivative may be 72% or more.

According to one embodiment, the enantiomeric excess (EE) of the α-amino acid derivative may be 90% or more.

The deuteration reaction of the proline derivative (4a) gradually proceeded according to reaction time, and showed a yield of 95%, a deuteration level (DL) of 72%, and an enantiomeric purity of 98% after 4 hours. After 8 hours, it showed a yield of 94%, a deuteration level (DL) of 98%, and an enantiomeric purity of 97%.

In order to search for optimal conditions for deuterating the proline derivative (4a), various bases, solvents, and reaction conditions were used.

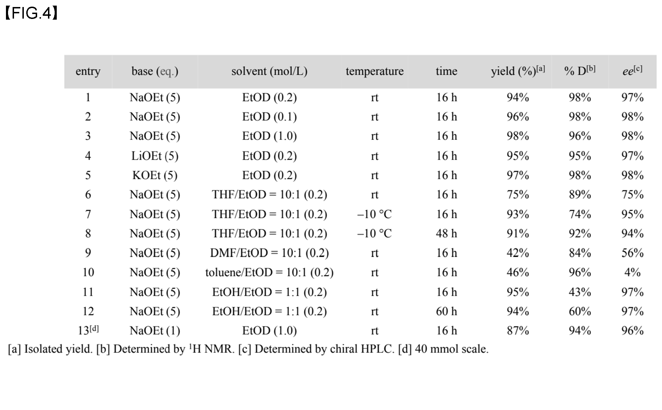

FIG. 4 is a table illustrating results of performing a deuteration reaction of a proline derivative under various conditions.

It was confirmed that the concentration of solvent EtOD did not have a great influence on the reaction result (entries 2, 3), and it was confirmed that the cation did not have a great influence on the reaction result (entries 4, 5) by using the same base in which only the cation was changed.

It was confirmed that a change in the composition of the solvent had an influence on the reaction result. When tetrahydrofuran (THF) was mixed with EtOD (10:1) and used together, the yield and enantiomeric purity were reduced compared to when only the EtOD solvent was used (entry 6), but when the reaction was performed at -10°C, the yield and enantiomeric purity were similar, but the reaction time was lengthened to 48 hours (entry 7). Through the corresponding result, the possibility of reducing the amount of EtOD used by mixing and using a relatively inexpensive solvent (THF) was confirmed. When dimethylformamide (DMF), which is a polar solvent, was mixed together (10:1) and used, the reaction efficiency was greatly reduced (entry 8). Even when toluene, which is a nonpolar solvent, was mixed together (10:1) and used, the yield and enantioselectivity were greatly reduced (entry 9). When EtOH was mixed with EtOD (1:1) and used together, the yield and enantiomeric excess were similar to when only EtOD was used, but the deuteration level was greatly reduced compared to when only EtOD was used (entries 10, 11).

Through the above results, it was confirmed that a high yield, deuteration level, and enantiomeric excess can be obtained when EtOD is used as the solvent. In addition, the above deuteration reaction was performed at room temperature, and thus it could be confirmed that a high deuteration level and enantiomeric purity can be obtained without extreme conditions.

### Experimental Embodiment 2: Mechanism analysis of deuteration reaction of proline derivative

Based on the above-described results, a mechanism in which a deuteration reaction occurs through a chiral enolate of the proline derivative as an intermediate in an EtOD solvent was analyzed. A density functional theory (DFT)-based computational investigation was performed using M06-2X functional with the 6-311++G(d,p).

Through computational conformation analysis, it was confirmed that, among 10 conformers of the proline derivative, the most stable conformer was 4a-I, and the 4a-I structure was set as a starting point of computer analysis.

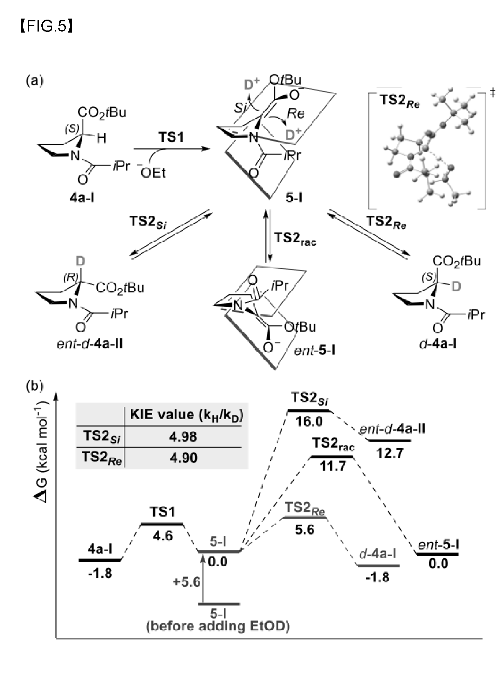

FIG. 5 is formula (a) illustrating a plausible reaction pathway of the asymmetric α-deuteration method for the proline derivative and a graph (b) illustrating a free energy profile.

When the proline derivative is deuterated in the conformer of 4a-I, a chiral enolate 5-I having a free energy barrier (ΔG) of 6.4 kcal/mol (TS1) was formed, and the enolate may have a change in chirality through several reaction pathways.

The free energy barrier (ΔG) of a pathway in which *d*-4a-I is formed from 5-I through TS2_{Re} was only 5.6 kcal/mol. On the other hand, the energy barrier of a pathway through TS2_{Si} was high at 16.0 kcal/mol, and the formed *ent-d*-4a-II was less stable. In addition, although *ent*-5-I generated through TS2_{rac} having an energy barrier of 11.7 kcal/mol had the same stability as the reactant 4a-I, it is not preferred due to a higher energy barrier. Through the above results, it could be expected that the pathway of TS2_{Re} forming *d*-4a-I would be the most preferred among several pathways through which 5-I can pass.

Entropic penalties generated by interaction between a substrate and EtOD in 5-I were analyzed by calculating each free energy. A free energy penalty of the substrate and EtOD for the deuteration reaction was 5.6 kcal/mol, and an overall free energy (ΔG) was analyzed as 11.2 kcal/mol for TS2_{Re}, 17.3 kcal/mol for TS2_{rac}, and 21.6 kcal/mol for TS2_{Si}.

When the kinetic isotope effect (KIE) of TS2_{Re} and TS2_{Si} was investigated, it was confirmed that a calculated reaction rate for deuterium substitution was 4.9 times to 5 times slower than a reaction rate for proton substitution. Therefore, it could be confirmed that the computer analysis results also coincided with the experimental results for the deuteration reaction.

### Experimental Embodiment 3: Analysis of deuteration reactions of various proline derivatives and amino acids

Results of deuteration reactions of various proline derivatives in which various kinds of protecting groups are substituted on N were analyzed. Various proline derivatives were treated with 5 equivalents (eq.) of NaOEt and 0.2 M EtOD at room temperature for 16 hours.

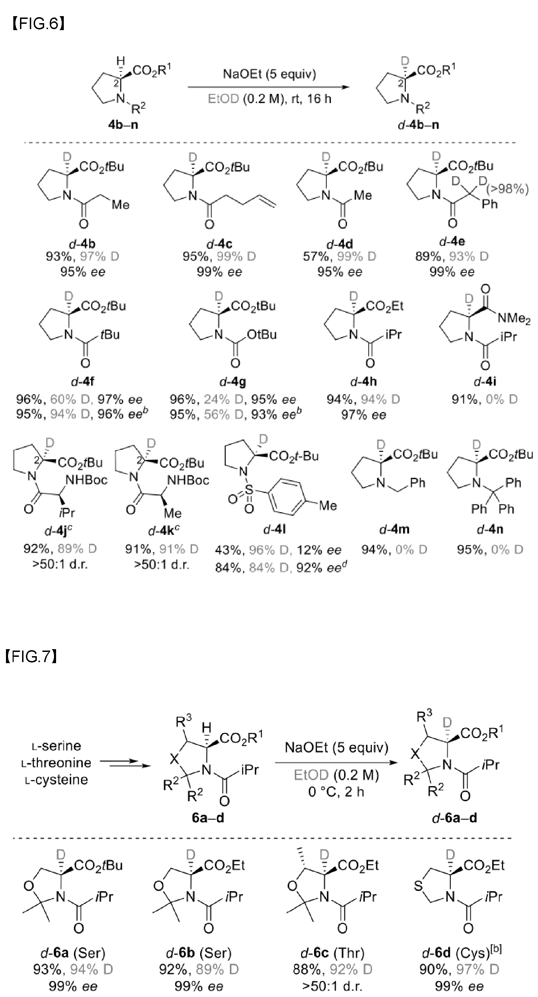

FIG. 6 is a drawing illustrating results of deuteration reactions of various proline derivatives.

*d*-4b and *d*-4c including a propionyl or pentenoyl group showed a high yield and enantiomeric excess. The derivative *d*-4e including a phenylacetyl group showed a high enantiomeric excess and deuteration level, and in this case, almost complete deuteration substitution was confirmed even at the α-position of the amide carbonyl group.

*d*-4f including a sterically bulky pivaloyl group as a protecting group showed only a deuteration level of 60% at a reaction time of 16 hours, but after 48 hours, the deuteration level increased to 94%, and the enantiomeric purity also reached 96%. *d*-4g including an *N*-Boc group showed a high yield and enantiomeric excess, and showed a low deuteration level of 24%, but the deuteration level after 48 hours increased to 56% without affecting the yield or enantioselectivity.

4h in which the *t*-butyl ester portion of 4a was changed to ethyl ester showed the same level of enantiomeric excess and deuteration level. On the other hand, 4i in which the *t*-butyl ester portion of 4a was changed to a dimethylamide group did not undergo deuteration due to a decrease in acidity of the C-2 hydrogen atom. As a result of deuteration reactions of dipeptides 4j (Boc-_{L}-Val-_{L}-Pro-O^{t}Bu) and 4k (Boc-_{L}-Ala-_{L}-Pro-O^{t}Bu), a high deuteration level was shown stereoselectively at the C-2 position. From the corresponding result, it is predicted that site-selective deuteration reaction at the C-terminal of a polypeptide would be possible.

In order to confirm the scope and limitations of substrates, when a deuteration reaction was performed with 4m and 4n in which an electron withdrawing group (EWG) was not included in the *N*-protecting group, it was confirmed that the deuteration reaction did not proceed. The deuteration reaction was also performed with 4l, to which a tosyl group, an electron withdrawing group (EWG) protecting group rather than a carbonyl *N*-protecting group, was applied. After 16 hours of the deuteration reaction at room temperature, a yield of 43%, a deuteration level of 96%, and an enantiomeric excess of 12% were shown. In order to improve this, 4l was subjected to the deuteration reaction under the same reagent conditions at -10°C for 48 hours, and a yield of 84%, a deuteration level of 84%, and an enantiomeric excess of 92% were shown. Through this, it could be confirmed that, in an enantioselective deuteration reaction, an electron withdrawing group (EWG) protecting group is essential for the N-protecting group, and among them, a protecting group including a carbonyl group is most effective.

In order to confirm whether the deuteration method of the present invention can also be applied to other α-amino acid derivatives, pseudoproline skeletons (6a-d) were synthesized using serine, threonine, and cysteine as starting materials, and the deuteration reaction was performed.

FIG. 7 is a drawing illustrating results of deuteration reactions of pseudoproline derivatives.

Deuteration of pseudoproline proceeded faster than proline derivatives at room temperature, but the yield was low due to instability. In order to improve this, deuteration reactions of 6a-d were performed at temperatures lower (0°C, -50°C for 4d) than room temperature, which is a basic condition. 6a and 6b, which are serine derivatives, showed results similar to those of the corresponding proline derivatives (4a, 4h), and threonine derivative 6c also showed high stereoselectivity with a yield of 88% and a diastereomer ratio of >50:1. Cysteine derivative 6d showed a high yield and deuteration level at room temperature, but was almost completely racemized, and thus the deuteration reaction was performed again by lowering the temperature (-50°C). Accordingly, the result was improved to a yield of 90%, a deuteration level of 97%, and an enantiomeric excess of 99%.

Following pseudoproline, results of deuteration reactions using acyclic α-amino acid derivatives as substrates were analyzed.

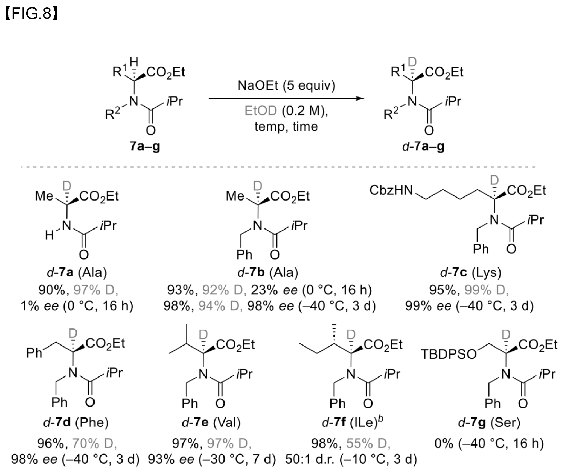

FIG. 8 is a drawing illustrating results of deuteration reactions of acyclic α-amino acid derivatives.

Alanine derivative 7a, into which only an isobutyryl group was introduced to a nitrogen atom, showed a high yield and deuteration level as a result of the deuteration reaction performed at 0°C, but was almost completely racemized. In order to improve this, alanine derivative 7b was synthesized by additionally introducing a benzyl group to the nitrogen atom, and the deuteration reaction was performed. Since an enantiomeric excess of 23% was shown as a result of the deuteration reaction at 0°C, in order to improve this, the reaction was performed for 3 days at -40°C by lowering the temperature, and results of a yield of 98%, a deuteration level of 94%, and an enantiomeric excess of 98% were obtained.

In a similar method, when lysine, phenylalanine, valine, and isoleucine derivatives (7c-f) were synthesized and the deuteration reaction was performed at a temperature of 0°C or lower, a high yield, deuteration level, and enantiomeric excess were obtained.

### Experimental Embodiment 4: Preparation of deuterated α-amino acid

A deuterated α-amino acid was prepared by removing a protecting group of the deuterated α-amino acid derivative prepared by the above method.

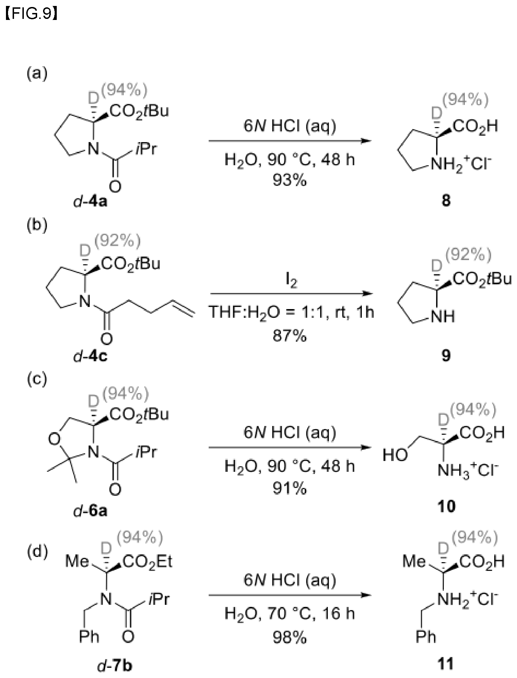

FIG. 9 is a drawing illustrating a step of removing an *N*-protecting group of a deuterated α-amino acid derivative.

6N HCl (1.0 mL) was added at room temperature to the deuterated proline derivative (*d*-4a) dissolved in water, and stirred at 90°C for 48 hours, followed by concentration under reduced pressure, thereby obtaining the deuterated α-amino acid (8) in a white solid state (14 mg, 93%). The deuteration level (DL) of the obtained α-amino acid (8) was 94%, and it was confirmed that it was the same as the deuteration level of *d*-4a before removing the protecting group. Through this, it could be confirmed that even when the *N*-protecting group is removed from the deuterated α-amino acid derivative, a deuterated α-amino acid can be obtained while maintaining chirality without loss of the deuteration level.

Similarly, it was confirmed that the deuterated α-amino acid derivative (9) prepared from the deuterated proline derivative (*d*-4c), the deuterated α-amino acid (10) prepared from the deuterated serine derivative (*d*-6a), and the deuterated α-amino acid derivative (11) prepared from the deuterated alanine derivative (*d*-7b) also showed the same deuteration level as before removing the protecting group.

From the above results, it could be confirmed that when the deuteration method of the present invention is used, in addition to a single α-amino acid derivative, deuteration in which chirality is maintained is also possible in a peptide compound in which a plurality of α-amino acids are connected. Therefore, it could be predicted that deuteration would be possible by the deuteration method even in larger-scale peptide and protein units.

The above description is merely an exemplary description of the technical idea of the present invention, and a person having ordinary skill in the technical field to which the present invention pertains will be able to make various modifications and variations without departing from the essential characteristics of the present invention. Therefore, the embodiments disclosed in the present invention are not intended to limit the technical idea of the present invention, but to explain the technical idea of the present invention, and the scope of the technical idea of the present invention is not limited by these embodiments. The protection scope of the present invention should be interpreted by the following claims, and all technical ideas within a scope equivalent thereto should be interpreted as being included in the scope of rights of the present invention.

## Claims

1. An asymmetric α-position hydrogen deuteration method of an α-amino acid derivative, comprising:
preparing an α-amino acid derivative including a structure of Chemical Formula 1;
treating the α-amino acid derivative with a base in a solvent to induce a deuteration reaction and generating a product; and
purifying the product. 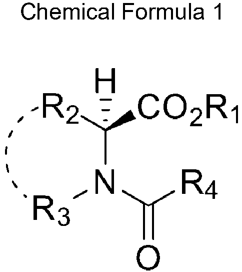
(R₁ includes one or more of alkyl, alkenyl, alkynyl, or hydrogen, R₂ and R₃ include one or more of alkyl, -C(O)alkyl, alkenyl, -C(O)alkenyl, cycloalkyl, -C(O)cycloalkyl, cycloalkenyl, -C(O)cycloalkenyl, aryl, -C(O)aryl, heteroaryl, -C(O)heteroaryl, heterocyclyl, or -C(O)heterocyclyl, R₄ includes one or more of alkyl, alkenyl, alkynyl, or hydrogen)

2. The asymmetric α-position hydrogen deuteration method of an α-amino acid derivative of claim 1,
wherein the method is substituting hydrogen at the α-position with deuterium while maintaining chirality at the α-position of the α-amino acid derivative.

3. The asymmetric α-position hydrogen deuteration method of an α-amino acid derivative of claim 1,
wherein the α-amino acid derivative includes an N-protecting group substituted on N.

4. The asymmetric α-position hydrogen deuteration method of an α-amino acid derivative of claim 1,
wherein the solvent includes one or more selected from the group consisting of a C₁ to C₁₀ alcohol-based solvent, acetonitrile, dimethyl sulfoxide (DMSO), acetone, ether and cyclic ether, nitromethane, chloroform, dimethylformamide (DMF), toluene, and water (H₂O), and
wherein the solvent includes at least one deuterium atom in a molecular structure to promote the deuteration reaction.

5. The asymmetric α-position hydrogen deuteration method of an α-amino acid derivative of claim 1,
wherein the base includes a cation and alkoxide (MOR).

6. The asymmetric α-position hydrogen deuteration method of an α-amino acid derivative of claim 1,
further comprising, between the step of generating the product; and the step of purifying the product;
stopping the deuteration reaction,
wherein the step of stopping the deuteration reaction is treating the product with a weakly acidic solution.

7. The asymmetric α-position hydrogen deuteration method of an α-amino acid derivative of claim 1,
wherein the step of purifying the product is purifying the product through one or more methods selected from the group consisting of chromatography, crystallization, extraction, distillation, recrystallization, filtration, evaporation, sublimation, fractional extraction, washing, and the like.

8. The asymmetric α-position hydrogen deuteration method of an α-amino acid derivative of claim 1,
further comprising, after the step of purifying the product;
removing an N-protecting group of the deuterated α-amino acid derivative included in the purified product.

9. An α-amino acid derivative including deuterium at the α-position,
wherein the α-amino acid derivative includes a structure of Chemical Formula 2, and
wherein Chemical Formula 2 is prepared by the asymmetric α-deuteration method of an α-amino acid derivative of claim 1. 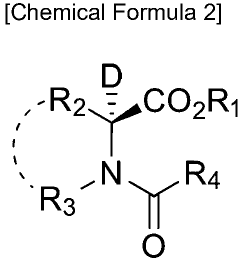
(R₁ includes one or more of alkyl, alkenyl, alkynyl, or hydrogen, R₂ and R₃ include one or more of alkyl, -C(O)alkyl, alkenyl, -C(O)alkenyl, cycloalkyl, -C(O)cycloalkyl, cycloalkenyl, -C(O)cycloalkenyl, aryl, -C(O)aryl, heteroaryl, -C(O)heteroaryl, heterocyclyl, or -C(O)heterocyclyl, R₄ includes one or more of alkyl, alkenyl, alkynyl, or hydrogen)

10. The α-amino acid derivative including deuterium at the α-position of claim 9,
wherein the α-amino acid derivative is a proline derivative in which hydrogen at the α-position is deuterated.

11. The α-amino acid derivative including deuterium at the α-position of claim 9,
wherein a deuteration level (DL) of the α-amino acid derivative is 72% or more.

12. The α-amino acid derivative including deuterium at the α-position of claim 9,
wherein an enantiomeric excess (EE) of the α-amino acid derivative is 90% or more.
